# EUROPEAN PATENT APPLICATION

(11) **EP 1 842 545 A1**
(43) Date of publication of application: **10.10.2007**
(21) Application number: 06450051.5
(22) Date of filing: 04.04.2006
(51) Int. Cl.: A61K 31/737, A61P 29/00, A61K 8/73

(54) **Anti-inflammatory polymer**

(71) Applicant: Marinomed Biotechnologie GmbH, 1210 Vienna (AT)
(72) Inventor: Grassauer, Dr., Andreas, 1220 Vienna (AT)
(74) Representative: Sonn & Partner Patentanwälte

(57) **Abstract**

The present invention provides the use of cellulose sulfate for the manufacture of an anti-inflammatory pharmaceutical or cosmetic composition for the treatment of inflammation.

## Description

The present invention relates to the field of immunology and anti-inflammatory agents.

Anti-inflammatory medicaments can be classified into those of steroid and of nonsteroidal type. Steroid anti-inflammatory compounds are still the most effective ones in the treatment of inflammatory diseases and conditions such as: asthma, chronic obstructive pulmonary disease, inflammatory nasal diseases such as allergic rhinitis, nasal polyps, intestinal diseases such as Crohn's disease, colitis, ulcerative colitis, dermatological inflammations such as eczema, psoriasis, allergic dermatitis, neurodermatitis, pruritis, conjunctivitis and rheumatoid arthritis. In addition to excellent potency and effectiveness, medicaments of this type also possess numerous unfavourable side-effects, (e.g. disturbance of carbohydrate metabolism, decreased calcium resorption, decreased excretion of endogenous corticosteroids and disturbance of physiological functions of the pituitary gland, adrenal cortex and thymus.

Psoriasis is a common skin disease characterized by hyperplasia of keratinocytes resulting in thickening of the epidermis and the presence of red scaly plaques. The lesions in this chronic disease typically are subject to remissions and exacerbations. There are several patterns, of which plaque psoriasis is the most common. Guttate psoriasis, with raindrop shaped lesions scattered on the trunk and limbs, is the most frequent form in children, while pustular psoriasis is usually localized to the palms and soles. The classical inflammatory lesions vary from discrete erythematous papules and plaques covered with silvery scales, to scaly itching patches that bleed when the scales are removed. Despite a voluminous scientific literature and numerous treatment strategies, there is still no effective treatment for psoriasis that is completely without side effects.

The number of different and sometimes toxic treatments employed for amelioration of psoriasis is testimony to the resistant nature of this disease. The devastating nature of this disease is emphasized by the extent of the side effects that psoriasis sufferers are willing to endure to attain a remission to a disease that they know will recur sooner or later.

It is a goal of the present invention to provide anti-inflammatory agents for the treatment of various diseases, especially skin diseases including psoriasis, related to immunoreactivities.

The present invention provides the use of a polymer for the manufacture of an anti-inflammatory pharmaceutical or cosmetic composition for the treatment of inflammation, wherein the polymer is cellulose sulfate. Cellulose sulfate as such is known in the state of the art. The manufacture of cellulose sulfate is e.g. disclosed in the DD 299313. As disclosed in US 2003/181415 A sulfated polysaccharides, including cellulose sulfate, are known to be effective against various enveloped viruses and in particular herpes simplex virus (HSV), Papilloma viruses and HIV. The preparation disclosed therein is used to prevent sexually transmitted infections. It is believed that cellulose sulfate binds to the lipid membrane of encapsulated viruses and prevents fusion to and infection of host cells.

It has now been demonstrated herein that cellulose sulfate shows significant anti-inflammatory action over cellulose, which has no immuno-modulating effects. It was completely surprising that such an effect could be achieved through sulfatation of cellulose. E.g. in the US 2006/0040896 A a heparin (a sulfated carbohydrate polymer of the glycosaminoglycan family, obtainable from liver) derivative is disclosed, wherein the anti-inflammatory efficacy was increased by desulfation.

Also disclosed herein is the use of chitosan as anti-inflammatory polymer. Chitosan is a linear polysaccharide composed of randomly distributed β-(1-4)-linked D-glucosamine (deacetylated unit) and N-acetyl-D-glucosamine (acetylated unit). Chitosan is produced commercially by deacetylation of chitin, which is the structural element in the exoskeleton of crustaceans (crabs, shrimp, etc.). There is a great diversity among chitosan products depending on the degree of deacetylation and origin of chitin. Most chitosan preparations are hardly soluble in neutral pH conditions. However, especially preferred is the use of water soluble chitosan. The production of water soluble chitosan is not trivial, but has already been demonstrated by Jakwang Co Ltd (KR 2001/027073, KR 2003/079444, US 2002/155175), which is a commercial supplier for water soluble chitosan. Water soluble chitosan is preferably completely soluble without the formation of colloids or gelatinous phases. Preferably the polymer is >90%, in particular preferred > 99% soluble (of its initial weight). Specifically preferred chitosan preparations, but also the general polymer preparations, are purified or purifyable by passage through a 0,22 µm pore sterilisation filter.

Without being limited to a specific theory it is assumed that the anti-inflammatory polymer of the present invention, e.g. cellulose sulfate binds to (and antagonizes) specific receptors of immune cells and prevents TNF-alpha production or release. TNF-alpha acts as activator for T-cells which in turn produce IFN-gamma. This is of particular significance in patients suffering from sepsis, where the TNF-gamma production is continuously triggered by bacterial antigens, which eventually leads to the septic shock syndrome, multiple organ failure and death.

Therefore in a preferred embodiment of the present invention the polymer is purified, in particular sterilely purified, e.g. through filtration through a sterilisation filter in soluble form.

In a preferred embodiment the pharmaceutical composition is for the treatment of inflammation of the skin or mucosa. The pharmaceutical preparation comprising the polymer is in particular used for the treatment of the following conditions involving inflammation of the skin or mucosa, e.g. that can be associated with persistent bacterial infections of the skin. These conditions include but are not limited to psoriasis, atopic dermatitis, neurodermitis, bullos diseases, Folliculitis, Erysipelas - St. Anthony's Fire, Hidradenitis Suppurativa, Rocky Mountain Spotted Fever - RMSF, Cuts, Scrapes, and Stitches involving bacterial Infection, Cutaneous Anthrax, Botulism, Plague, Tularemia, Skin Abscesses, Carbuncles, Cat Scratch Disease, Cellulitsis (superficial bacterial infection of the skin), Erysipelas, Furuncles, Furunculosis, Hidradenitis Suppurativa, Folliculits, Impetigo, Staphylococcus aureus, Methicillin resistant Staph. Aureus, Staphylococcal scalded skin syndrome, Toxic shock syndrome, Streptococcus pyogenes, Necrotising fasciitis, Scarlet fever, Gonorrhoea, Meningococcal disease, Erysipelothrix insidiosa, Haemophilus ducreyi, Haemophilus (cause of cellulitis in young children), Klebsiella rhinoscleromatis, Pseudomonas aeruginosa, Calymmatobacterium granulomatis, Treponema species cause syphilis, yaws and pinta, Borrelia species cause Lyme disease, Mycobacterium species cause tuberculosis, leprosy and atypical mycobacterial infections. Other conditions sometimes caused by bacterial infection include, Kawasaki disease (mucocutaneous lymph node syndrome), Pseudofolliculitis barbae (shaving bumps), Sarcoidosis, Scalp folliculitis, uticaria, Conjunctivitis, sepsis (e.g. septic shock) and rheumatism.

In another preferred embodiment the pharmaceutical composition is for the treatment of complete or partial autoimmune etiology. In particular, the pharmaceutical composition is used for the treatment the following diseases with a complete or partial autoimmune etiology:
- Coeliac disease is a disease characterised by chronic inflammation of the proximal portion of the small intestine caused by exposure to certain dietary gluten proteins;
- Crohn's disease is a form of inflammatory bowel disease characterized by chronic inflammation of the intestinal tract. Major symptoms include abdominal pain and diarrhea;
- Lupus erythematosus is a chronic (long-lasting) autoimmune disease wherein the immune system, for unknown reasons, becomes hyperactive and attacks normal tissue;
- Pemphigus is an autoimmune disorder that causes blistering and raw sores on skin and mucous membranes;
- Psoriasis is a skin disorder in which rapidly-multiplying skin cells produce itchy, scaly inflamed patches on the skin;
- Reiter's syndrome is an autoimmune attack on various body systems in response to a bacterial infection and the body's confusion over the HLA-B27 marker;
- Temporal arteritis (also known as "giant cell arteritis") is an inflammation of blood vessels, most commonly the large and medium arteries of the head. Untreated, the disorder can lead to significant vision loss.

Preferably the pharmaceutical preparation is in form of a preparation for topical or mucosal use, preferably skin lotions, cremes, sprays or gargle solutions. The polymer is especially suitable for topical application to treat skin or mucosal inflammation. But also systemic, e.g. parenteral or oral (also for specific mucosal treatment), is possible, especially for low molecular weight polymers. The present invention also provides the use of the pharmaceutical preparations.

Preferably, the polymer has a molecular weight ranging from about 15000 to 3,000,000 Da. Preferably, the molecular weight is greater than about 500,000, or for systemic administration lower than 500,000 Da.

Also preferred is that the preparation comprises pharmaceutical carriers or additives. The term "carrier" refers to a diluent, e.g. water, saline, excipient, or vehicle with which the composition can be administered. For a solid or fluid composition the carriers or additives in the pharmaceutical composition may comprise SiO₂, TiO₂, a binder, such as microcrystalline cellulose, polyvinylpyrrolidone (polyvidone or povidone), gum tragacanth, gelatine, starch, lactose or lactose monohydrate, alginic acid, maize starch and the like; a lubricant or surfactant, such as magnesium stearate, or sodium lauryl sulphate; a glidant, such as colloidal silicon dioxide; a sweetening agent, such as sucrose or saccharin. Preferably the preparation comprises buffers or pH adjusting agents, e.g. selected from citric acid, acetic acid, fumaric acid, hydrochloric acid, malic acid, nitric acid, phosphoric acid, propionic acid, sulfuric acid, tartaric acid, or combinations thereof. Cellulose sulfate in the form of a pharmaceutically acceptable salt, for example sodium cellulose sulfate may also be used. Other pharmaceutically acceptable salts include, among others, potassium, lithium and ammonium cellulose sulfate.

The degree of sulfation of cellulose sulfate is preferably above 12% and most preferably about 17-18%, or maximal sulfation.

The polymer is preferably a homopolymer, e.g. pure cellulosesulfate, or alternatively a derivative heteropolymer.

Furthermore, preferably the polymer is in amounts between 0,01% and 20%, preferably between 0,1% and 10%, most preferred between 0,5% and 5% of the preparation (%-values are given in w/w-%). In the pharmaceutical preparation also other anti-inflammatory polymers than cellulose sulfate can be present, most preferably water soluble chitosan. For these polymers the same preferred concentration ranges apply.

The administration of the preparation is not limited to administrations at the same time of inflammation but can also be used before or after an inflammation, e.g. for prophylactic treatment, i.e. a treatment before an expected inflammation to reduce the force of the inflammation.

In the preparation only one anti-inflammatory agent, e.g. cellulose sulfate, may be present. Nevertheless, in a more preferred embodiment the pharmaceutical preparation comprises at least two different anti-inflammatory components, preferably one component is chitosan. As has been shown herein (examples) chitosan, especially water soluble chitosan (Jakwang), is an exceptional anti-inflammtory polymer (alone or in combination), which can be used in a preparation of the present invention.

The present invention is further illustrated by the following Figures and examples.

### Figures:

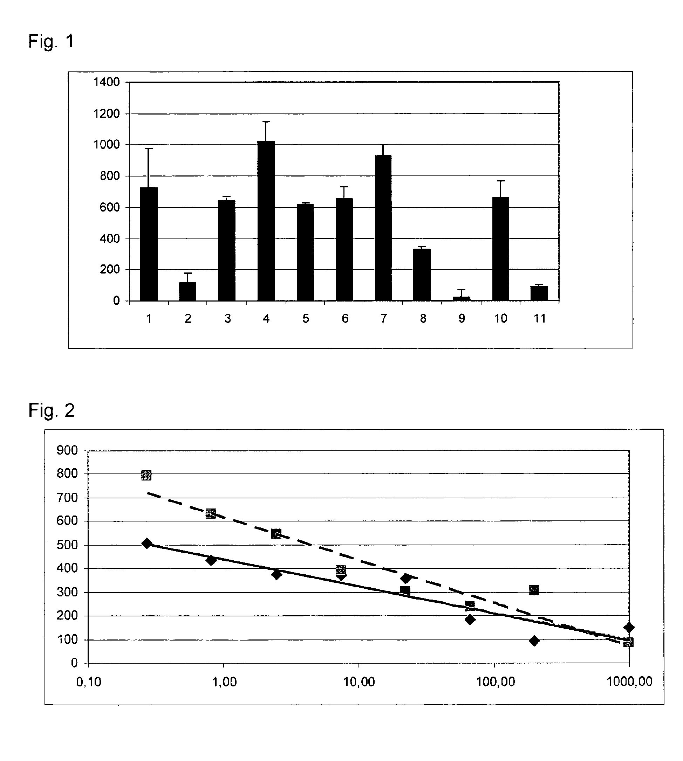

Figure 1: LPS induced TNF-alpha production is inhibited by Cellulose sulphate and water soluble Chitosan but not other biopolymers.
   The y-axis shows the amount of TNF-alpha in pg/ml that is produced after 18hours of incubation with LPS and the corresponding biopolymer. The black bars correspond to a test substance concentration of 330µg/ml. The numbers at the x-axis indicate the corresponding Biopolymers whereas 1 is Carboxymethylcellulose T70 (Niklacell, Mare GmbH, Austria), 2 is water soluble Chitosan (Jakwang, Korea), 3 Carboxymethylchitosan, 4 is Carrageenan (Carbamer), 5 is Carboxymethylcellulose T35 (Niklacell, Mare GmbH, Austria), 6 is kappa Carrageenan (Sigma), 7 is lambda Carrageenan (Sigma), 8 is cellulose sulfate (Acros), 9 uninduced not treated blood, 10 LPS induced blood and 11 is the Dexamethasone (Sigma) reference control 100ng/ml.
Figure 2: The inhibition of TNF-alpha production by Cellulose sulphate and Chitosan is dose dependent
   The y-axis shows the amount of TNF-alpha in pg/ml produced after 18hours of incubation with LPS and the corresponding biopolymer. The X-axis shows the test substance concentration in µg/ml at a logarithmic scale. Cellulose sulphate data is shown in diamonds and the corresponding fit as black line. Chitosan is shown as grey squares and the fit as dashed line.

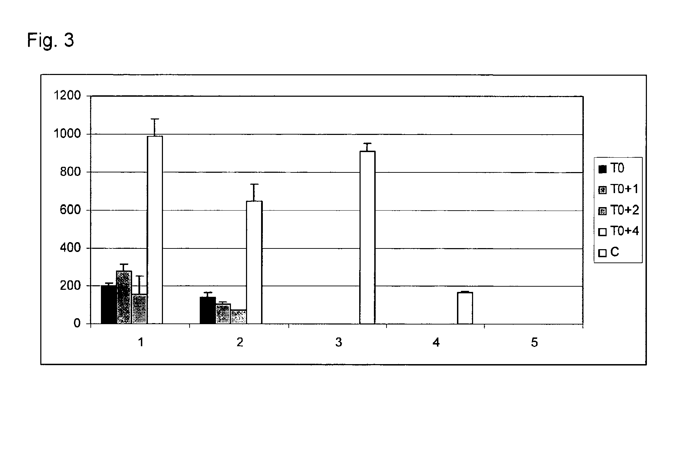

Figure 3: Cellulose Sulphate and Chitosan are active by administration hours after stimulation
   The y-axis shows the amount of TNF-alpha in pg/ml produced after 18hours of incubation with LPS and the corresponding biopolymer at different time points. The black bars indicate the addition of the test substance at the same time with LPS. For grey bar data the test substance was added 1h after the stimulus, dashed bars 2 hours after stimulus and white bars 4 hours after stimulus. Lane number 1 on the x-axis corresponds to Cellulose sulphate and number 2 to chitosan both at a concentration of 20µg/ml. The wave shaped bars in lanes 3, 4 and 5 indicate the assay controls which are indicated as with LPS stimulus without substance, LPS + Dexamethasone reference substance and unstimulated blood respectively.

### Examples:

A number of polymers were compared for their ability to inhibit the LPS induced stimulation of whole blood as determined by the release of TNF alpha. Cellulose sulphate and high molecular weight chitosan inhibited the LPS induced TNF-alpha production. In contrast, a series of other biopolymers did not have an effect. In addition, cellulose sulphate is effective when it is administrated even 2 hours after the stimulus.

### Example 1: Materials and Methods

The inducing or inhibitory effects of cellulose sulfate and other biopolymers were investigated using primary blood cells in combination with cytokine ELISAs such as TNF-alpha. The cells were either left non-stimulated or they were induced with 200 ng/ml LPS (Lipopolysaccharide). The cells were stimulated for 18h and TNF-alpha secretion was measured by using a commercial TNF-alpha ELISA (R&D systems). The results are given in figs. 1-3.

As it can be seen in Figure 1 Chitosan (2) and cellulose sulphate (9) have a TNF-alpha inhibition activity. Other polymers do not have a significant inhibiting activity.

As shown in Figure 2 the TNF-alpha inhibiting effect of Cellulose sulphate and Chitosan is dose dependent and the two polymers inhibit at comparable concentrations.

As shown in Figure 3 Cellulose Sulphate and Chitosan are active up to 2 hours after stimulation. 2 hours after stimulation both polymers were as active as when added at the same time with LPS. When the polymers are added 4hours after the stimulus the inhibitory effect is dramatically reduced.

### Example 2: Activity of cellulose sulphate and other bioploymers on immune cells

Cellulose sulfate was investigated concerning its potential activating or inhibiting function on primary human blood cells. Several experiments that measure the induction of cytokines were performed that suggest that cellulose sulfate on its own is not immunostimulatory neither on blood cells nor on isolated lymphocytes. In contrast, cellulose sulfate markedly reduced the production of the pro-inflammatory cytokine TNF-alpha in LPS stimulated primary blood cells. Therefore, it is concluded that cellulose sulfate acts as a novel anti-inflammatory substance.

In an analogous experiment the immuno-stimulatory capacity of cellulose sulfate was determined. Cells were either left non-stimulated, activated with PMA + ionomycin or LPS or treated with 2 different batches of cellulose sulfate. Both batches did not lead to any induction of TNF-alpha release. In a similar setting also the cytokines IL-1 beta, IL-6, and IL-12 p40 were tested with identical results. Primary lymphocytes were tested in a similar setting for the capacity of cellulose sulfate to induce the release of IL-2 or IFN-gamma. Again no cytokine stimulation was detected.

Taken together the data indicate that cellulose sulfate does not provoke a cytokine release of primary blood cells or primary lymphocytes in-vitro, but exhibits a remarkable inhibitory effect on LPS stimulated primary blood cells.

## Claims

1. Use of cellulose sulfate for the manufacture of an anti-inflammatory pharmaceutical or cosmetic composition for the treatment of inflammation.

2. Use according to claim 1, **characterized in that** the pharmaceutical composition is for the treatment of inflammation of the skin or mucosa.

3. Use according to claim 1 or 2, **characterized in that** the pharmaceutical composition is for the treatment of conditions selected from psoriasis, atopic dermatitis, neurodermitis, bullos diseases, folliculitis, erysipelas, hidradenitis suppurativa, Rocky Mountain Spotted Fever, bacterial infected cuts, scrapes and stitches, cutaneous anthrax, botulism, plague, tularemia, skin abscesses, carbuncles, Cat Scratch Disease, cellulitsis, erysipelas, furuncles, furunculosis, hidradenitis suppurativa, folliculits, impetigo, staphylococcus aureus, in particular methicillin resistant staphylococcus aureus, staphylococcal scalded skin syndrome, toxic shock syndrome, streptococcus pyogenes, necrotising fasciitis, scarlet fever, gonorrhoea, meningococcal disease, erysipelothrix insidiosa, haemophilus ducreyi, haemophilus, klebsiella rhinoscleromatis, pseudomonas aeruginosa, calymmatobacterium granulomatis, treponema, inparticular in syphilis, yaws and pinta, borrelia, in particular in lyme disease, mycobacterium, in particular in tuberculosis, leprosy, atypical mycobacterial infections, Kawasaki disease, pseudofolliculitis barbae, sarcoidosis, scalp folliculitis, uticaria, conjunctivitis, sepsis and rheumatism.

4. Use according to claim 1, **characterized in that** the pharmaceutical composition is for the treatment of complete or partial autoimmune etiology.

5. Use according to claim 1 or 4, **characterized in that** the pharmaceutical composition is for the treatment of Coeliac disease, Crohn's disease, Lupus erythematosus, Pemphigus, Psoriasis, Reiter's syndrome or temporal arteritis ("giant cell arteritis").

6. Use according to any one of claims 1 to 5, **characterized in that** the preparation is in form of a preparation for topical or mucosal use, preferably skin lotions, cremes, sprays or gargle solutions.

7. Use according to any one of claims 1 to 6, **characterized in that** the preparation comprises pharmaceutical carriers or additives.

8. Use according to any one of claims 1 to 7, **characterized in that** the polymer is in amounts between 0,01% and 20%, preferably between 0,1% and 10%, most preferred between 0,5% and 5% of the preparation.

9. Use according to any one of claims 1 to 8, **characterized in that** the preparation comprises at least two different anti-inflammatory components, preferably one component being chitosan.

10. Use according to any one of claims 1 to 9, **characterized in that** the preparation is sterile.
